# EUROPEAN PATENT APPLICATION

(11) **EP 4 520 362 A1**
(43) Date of publication of application: **12.03.2025**
(21) Application number: 23306504.4
(22) Date of filing: 08.09.2023
(51) Int. Cl.: A61M 5/00, A61B 50/30, A61L 2/00, A61L 2/07, A61L 2/08, A61L 2/20, B65B 55/02

(54) **STERILE DOOR COVER FOR MEDICAL STOPPER TRANSFER**

(71) Applicant: Becton, Dickinson and Company, Franklin Lakes, NJ 07417-1880 (US)
(72) Inventor: LE LOC'H, Clémentine, 38240 Meylan (FR); EYMERY, Anaïs, 38450 Saint-Georges-de-Commiers (FR)
(74) Representative: Germain Maureau

(57) **Abstract**

A container is disclosed, including a bag defining a first end, a second end, an exterior surface, and an interior cavity; a connector defining a first end, a second end, and a passage therethrough, wherein the first end of the connector is attached to the first end of the bag, and the passage is in fluid communication with the interior cavity; and a cover attached to at least one of the bag and the connector, wherein the cover is transitionable from a first configuration where the cover surrounds at least a portion of the exterior surface of the bag, to a second configuration where the cover encloses the second end of the connector.

## Description

### FIELD OF THE INVENTION

The present disclosure relates generally to the packaging and transfer of sterile components used, for example, in medical devices. More particularly, the present disclosure relates to containers with protective covers, uses, and methods of manufacture thereof for the transfer of sterilized components.

### BACKGROUND OF THE INVENTION

As is known in the art, transfer or storage devices for delivery of a medicament, drug, or vaccine (such as, e.g., syringes) utilize a plunger stopper in contact with an inside surface of a generally tubular syringe barrel in order to draw a substance into (or expel a substance from) the device by way of a plunger rod.

Currently, many such devices are filled and assembled using automated filling machines. Not only do such machines improve productivity and accuracy, but they also provide for a substantially sterile and aseptic filling environment. The various components of the devices (e.g., plunger stoppers, syringe barrels, etc.) are separately provided within the filling machines to enable at least some level of automated assembly.

Generally, a plurality of plunger stoppers (or other sterilizable device components) are initially provided in a substantially flexible bag or similar container to be accessed by the filling machine prior to assembly. The bag generally has a connector configured for connecting to a transfer port of an automated filling machine. The bag and its contents are sterilized via, e.g., gamma irradiation, steam, etc. In this way, the plunger stoppers, are able to be directly transferred from a first sterile environment (i.e., the sterilized bag) through the connector to a second sterile environment (i.e., the sterile filling machine).

The connector and passageway therethrough into the interior of the bag are generally protected with a protective cover to prevent contamination of the passageway and sterilized bag contents during shipping and handling prior to connection with the sterile filling machine. Generally, the protective cover is a fabric cover having an elastic band that is configured to fit around the connector. There are several disadvantages associated with existing protective covers for transfer bags. The fabric material of the protective cover is opaque, thereby preventing visual verification of the integrity of the connector and/or passageway. In order for the user to verify the integrity of the connector, the protective cover must be temporarily removed, thereby exposing the connector and interior to undesired contamination. The elastic band of the protective cover can be installed in multiple positions relative to the connector, leading to low repeatability of preferred installation. Moreover, the elastic fitting can render the cover susceptible to shifting or inadvertent removal, thereby exposing the connector and bag contents to potential contamination.

### SUMMARY OF THE INVENTION

The present disclosure provides a container, including a bag defining a first end, a second end, an exterior surface, and an interior cavity; a connector defining a first end, a second end, and a passage therethrough, wherein the first end of the connector is attached to the first end of the bag, and the passage is in fluid communication with the interior cavity; and a cover attached to at least one of the bag and the connector, wherein the cover is transitionable from a first configuration where the cover surrounds at least a portion of the exterior surface of the bag, to a second configuration where the cover encloses the second end of the connector.

The bag may be made of at least one of polyethylene (PE), high-density polyethylene (HDPE), thermoplastic elastomer (TPE), polypropylene, polyvinylidene fluoride (PVDF). The cover may be made of at least one of polyethylene (PE), high-density polyethylene (HDPE), polypropylene (PP), polyvinylidene fluoride (PVDF). The connector may be made of at least one of polycarbonate (PC), acrylonitrile butadiene styrene (ABS), polyvinylidene fluoride (PVDF), polybutylene terephthalate (PBT). The bag may be attached to an inner perimeter of the connector. The cover may be attached to an exterior perimeter of the connector. The bag may be attached to an exterior perimeter of the first end of the connector. The cover may be attached to an exterior perimeter of the connector. The cover may be attached to an exterior perimeter of the first end of the bag.

The container may include an overmolding attached to at least a portion of the connector, and at least one of the cover and the bag may be attached to the overmolding. The overmolding may be made of at least one of polyethylene (PE) or polypropylene (PP). The cover may include a portion of the bag folded over itself. The cover may define a first end sealed to at least one of the bag and the connector, and the cover may define a second end independently movable about the connector.

A method for producing a container is disclosed, including providing a bag having a first end, a second end, and a cavity therein; forming a fold in the bag such that a first portion of the bag covers at least a portion of an exterior surface of a second portion of the bag; and securing at least a portion of the fold to a connector configured to be connected to a chamber, the connector defining a passage in fluid communication with the cavity of the bag. Forming the fold may include positioning the first portion of the bag to circumscribe at least a portion of the exterior surface of the second portion. The first portion of the bag may be sized to enclose the connector therein. The method may include manipulating the first portion of the bag to enclose the connector therein, and sealing the first portion of the bag. The method may include sterilizing the first and second portions of the bag.

A method of sterilizing one or more components is disclosed, including moving one or more components into an interior cavity of a bag attached to a connector; enclosing the connector within a cover, wherein the cover includes a first end sealed to the connector, and a second end independently movable about the connector; sealing the second end of the cover to contain the connector therein; and sterilizing the connector within the cover and the one or more components within the bag. Sterilizing the connector and the one or more components may be performed using at least one of ethylene oxide, gamma radiation, x-ray radiation, and/or may be performed using steam.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings, wherein:
FIG. 1 illustrates an example of a container with a cover in a first position in accordance with the principles of the present disclosure;
FIG. 2 illustrates the container of FIG. 1 with a cover in a second position in accordance with the principles of the present disclosure;
FIG. 3 illustrates an example of attaching of one or more components of a container in accordance with the principles of the present disclosure;
FIG. 4 illustrates another example of attaching of one or more components of a container in accordance with the principles of the present disclosure;
FIG. 5 illustrates another example of attaching of one or more components of a container in accordance with the principles of the present disclosure;
FIG. 6 illustrates another example of attaching of one or more components of a container in accordance with the principles of the present disclosure;
FIG. 7 illustrates an example of a bag in accordance with the principles of the present disclosure;
FIG. 8 illustrates an example of manipulating the bag of FIG. 7; and
FIG. 9 illustrates an example of a container with a connector constructed from the bag of FIGS. 7-8.

### DETAILED DESCRIPTION OF THE INVENTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As used herein, the singular form of "a", "an", and "the" include plural referents unless the context clearly dictates otherwise.

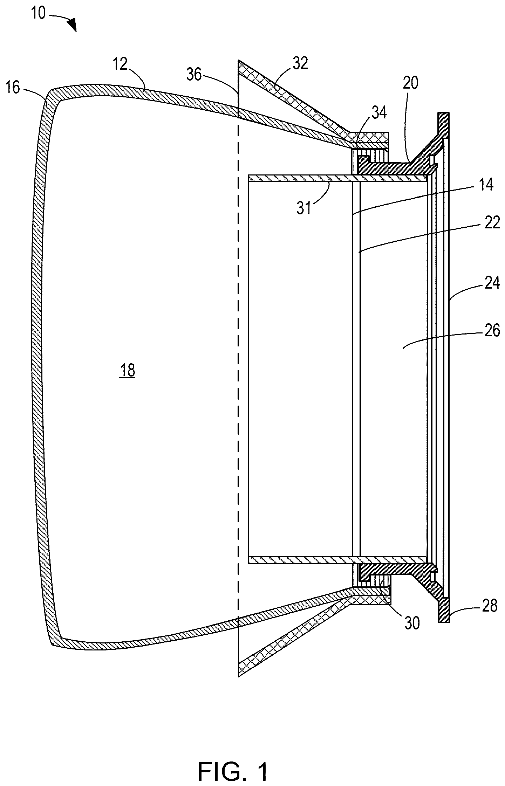

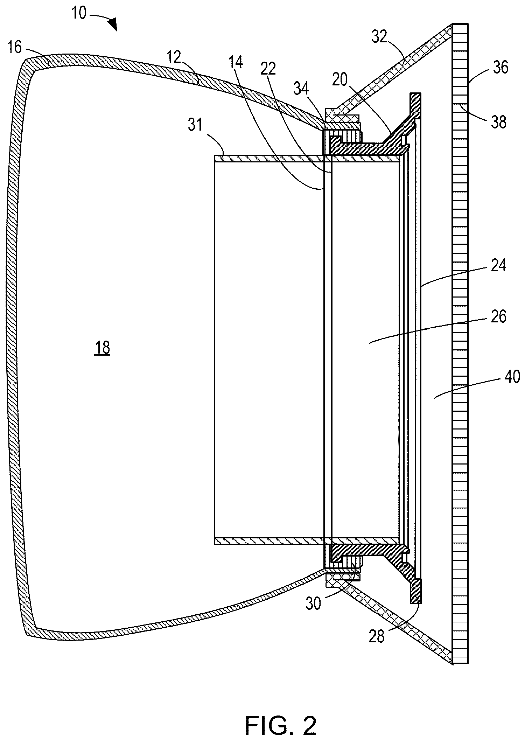

The present disclosure provides containers for packaging and transfer of sterile components, and methods of manufacture and uses thereof. Referring now to the drawing figures in which like reference designations refer to like elements, examples of a container 10 are shown in FIGS. 1-8. With reference to FIGS. 1 and 2, the container 10 is generally configured for storage and subsequent transfer of sterilized components from the container 10 into a sterile chamber of a processing machine through a transfer port. The container 10 includes a bag 12 having a first end 14, a second end 16, and an interior cavity 18. The bag 12 may be sized, shaped, and configured to hold a desired volume or quantity of sterilized or sterilizable components in the cavity 18 therein, such as, e.g., a plurality of plunger stoppers, a plurality of needle covers, a plurality of tip caps, tools, etc. The bag 12 may be formed of any appropriate flexible material suitable for steam sterilization, gamma sterilization, or ethylene oxide sterilization. Examples of such a flexible material may include, e.g., polyethylene (PE), high-density polyethylene (HDPE), polypropylene (PP), polyvinylidene fluoride (PVDF), etc.

Referring still to FIGS. 1 and 2, the container 10 further includes a connector 20 configured to securely connect the container 10 to a chamber, door, or other device for the transfer of contents into or out of the interior cavity 18 of the bag 12. The connector defines or includes a first end 22, second end 24, and a passage 26 extending therebetween. The first end 22 of the connector 20 may be attached or secured to the first end 14 of the bag 12 such that the passage 26 is in fluid communication with the interior cavity 18 of the bag 12. The second end 24 of the connector 20 may include a rim, flange, or other feature 28 that matably connects, locks, or otherwise engages a complementary feature on a door or passage of a chamber or device transferring contents in or out of the bag 12. The feature 28 enables or facilitates a sealed connection between the connector 20 (and thus the interior of the bag 12) and a chamber attached thereto.

The connector 20 is configured to support the locking or engagement forces against a door of a transfer portal of a sterile chamber, and accordingly, may be formed of any appropriate rigid material that is also suitable for steam sterilization, gamma sterilization, or ethylene oxide sterilization, such as, e.g., polycarbonate (PC), acrylonitrile butadiene styrene (ABS), polyvinylidene fluoride (PVDF), polybutylene terephthalate (PBT), etc.

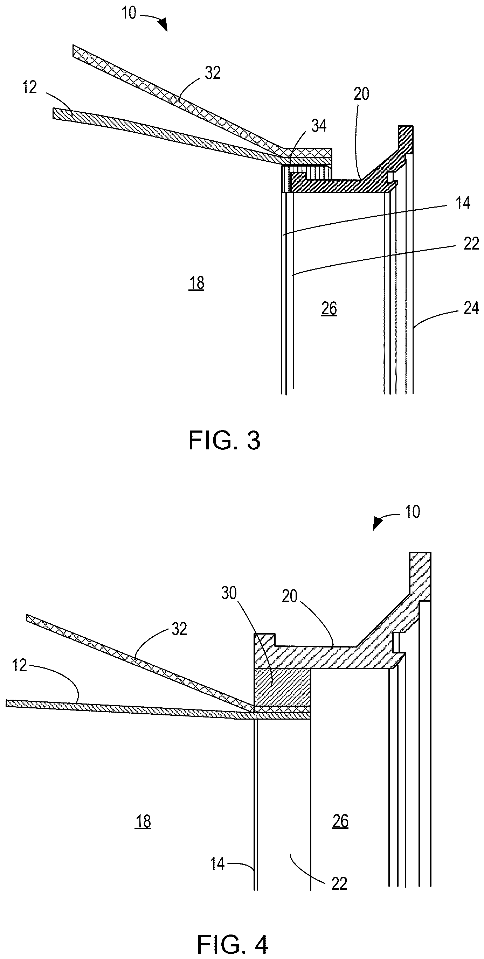

The container 10 may include an overmolding 30 on one or more surfaces of the connector 20. The overmolding 30 may provide desired tactile, structural, or attachment features in conjunction with the other components of the container 10, as described herein. In the example of the container 10 shown in FIGS. 1-3, the overmolding 30 extends around at least a portion of an outer perimeter of the first end 22 of the connector 20. The overmolding 30 may further extend along a length of a sidewall between the first end 22 and second end 24 of the connector 20. In an alternative configuration shown in FIG. 4, the overmolding 30 extends around at least a portion of an inner perimeter of the first end 22 of the connector 20, adjacent to, aligned with, and/or extending within the passage 24. The overmolding 30 may be formed of any material suitable for bonding to the components of the container 10, such as the bag 12 and/or connector 20, such as, e.g., polyethylene (PE), polypropylene (PP), thermoplastic elastomer (TPE), etc. In one non-limiting example or aspect, the overmolding 30 may be constructed from a material having the same or similar base polymer as the material constituting the bag 12 and/or cover 32 to enhance, improve, or facilitate bonding and sealing of these components.

The container 10 may include a sleeve or chute 31 connected to the connector 20 and extending into a portion of the interior cavity 18 of the bag 12. The sleeve 31 may be constructed from one or more materials similar to the bag 12. The sleeve 31 may have a generally cylindrical shape having a circumference or width similar to a width or circumference of the passage 26 to enable or facilitate directing contents through at least a portion of the passage 26.

The container 10 may include a cover 32 attached to the bag 12 and/or connector 20 to provide an additional sealed, sterilizable interior therein. For example, as shown in FIG. 1, the cover 32 may include a first end 34 attached at or in proximity to the first end 14 of the bag 12 and/or at or in proximity to the first end 22 of the connector 20. The cover 32 may include a second end 36 that is open and movable about at least a portion of the bag 12 and the connector 20. The cover 32 may be coaxial with and/or concentrically positioned with respect to the bag 12. The second end 36 of the cover 32 may be manipulated and configurable into a first position where at least a portion of the cover 32 surrounds or overlays at least a portion of an exterior surface of the bag 12, as shown in FIG. 1. The second end 36 of the cover 32 may also be manipulated and configurable into a second position where at least a portion of the cover 32 surrounds or encloses the second end 24 of the connector 20, as shown in FIG. 2. The second end 36 of the cover 32 may be closed, fused, or otherwise form a seal 38 to define an interior cavity 40 enclosing the connector 20 therein for sterilization and/or sterility maintenance. The interior cavity 40 may also provide pressure stabilization with the cavity 18 of the bag 12 to offset or balance fluctuations in internal pressure during transport or storage of the container 10. The seal 38 may be formed by any appropriate method of sealing such as, e.g., heat sealing, adhesive sealing, etc. The cover 32 may be formed of any appropriate flexible material (the same as or similar to the bag 12) suitable for steam sterilization, gamma sterilization, or ethylene oxide sterilization. Examples of such a flexible material may include, e.g., polyethylene (PE), high-density polyethylene (HDPE), polypropylene, polyvinylidene fluoride (PVDF), etc.

The bag 12 and cover 32 may be attached to the connector 20 by any appropriate method of sealing such as, e.g., heat sealing, adhesive sealing, laser welding, etc. The bag 12 and/or cover 32 may attach directly to the connector 20 and/or attach indirectly to the connector 20 through the overmolding 30. The bag 12 and cover 32 may be separately attached or sealed to different locations of the connector 20 and/or overmolding 30, or alternatively, may be overlaid, fused, welded, and/or otherwise sealed one on top of the other at one or more connection points on or to the connector 20 and/or overmolding 30. In one non-limiting example or aspect, the bag 12 and/or cover 32 may be contiguously sealed around a circumference or perimeter of the connector 20 to provide a substantially fluid-tight seal to maintain sterility of the interior portions of the container 10, as described herein.

In one non-limiting aspect or example, sealing the bag 12 and/or cover 32 to the connector 20 may be performed by rotating one or more welding, heating, or adhering devices (not shown) 360° around the bag 12 and/or cover 32 when mounted or positioned onto the connector 20. In another non-limiting example, one or more welding, heating, or adhering devices may be statically positioned or otherwise stationary while the bag 12 and/or cover 32 assembled with the connector 20 are collectively rotated with respect to the welding or heating devices to seal the components together.

The sequence of processes sealing the bag 12 and the cover 32 to the connector 20 may vary for particular applications or intended uses of the container 10. For example, the bag 12 and the cover 32 may be overlaid or adjacently positioned and sealed to the connector 20 in a single bonding or attachment process. In another example, the bag 12 may be sealed to the connector 20 in a first bonding or attachment process, and the cover 32 may be sealed to the connector 20 in a second bonding or attachment process (or vice versa). The first and second processes (and/or additional processes) may be performed or effected at the same location or at different locations with respect to the connector 20.

The location of the attachment(s) of the bag 12 and the cover 32 to the connector 20 may vary for particular applications or intended uses of the container 10. For example, as shown in FIG. 3, the bag 12 and/or the cover 32 may be attached to the connector 20 and/or or overmolding 30 around an exterior surface or perimeter of the first end 22.

In another example, as shown in FIG. 4, the bag 12 and/or the cover 32 may be attached to the connector 20 and/or overmolding 30 around an interior surface or perimeter of the first end 22. The sleeve 31 (not shown in FIG. 4) may also be overlaid or adjacently positioned to the bag 12 and/or the cover 32 on or around the interior surface or perimeter of the first end 22.

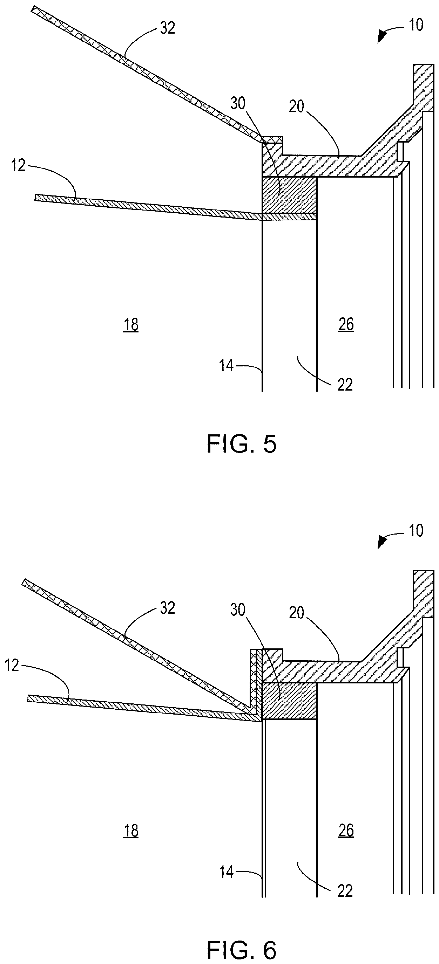

In yet another example, as shown in FIG. 5, the bag 12 may be attached to the connector 20 and/or overmolding 30 around an interior surface or perimeter of the first end 22, and the cover may be attached to the connector 20 and/or overmolding 30 around an exterior surface or perimeter of the first end 22.

In yet another example, as shown in FIG. 6, the bag 12 and/or the cover 32 may be attached to the first end 22 of the connector 20 oriented substantially perpendicular or transverse to an axis of the passage 26 passing through the connector 20 and/or a longitudinal axis of the container 10.

The bag 12 and cover 32 of the container 10 may be constructed or assembled from discrete and separate components, or alternatively, may be constructed and assembled from a single unitary receptacle formed from a flexible polymer. For example, the bag 12 may be initially provided separate from the cover 32, and the cover 32 may be concentrically or coaxially aligned about a portion of the exterior surface of the bag 12. The stacked or aligned cover 32 and bag 12 may then be positioned about an exterior or interior surface of the first end 22 of the connector 20, and the cover 32 and bag 12 may then be welded, molded, adhered, or otherwise sealed to the connector 20 to form the container 10 as generally shown in FIG. 1.

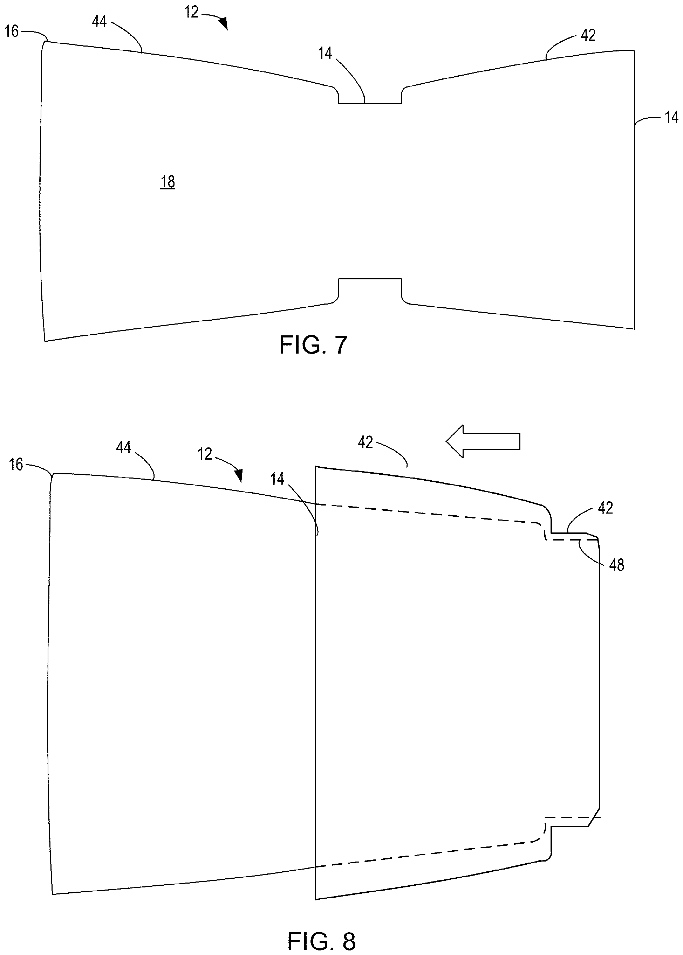

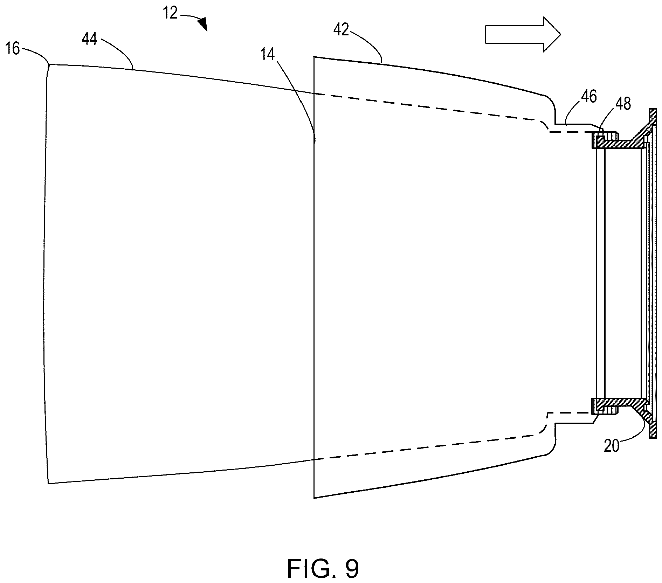

In another example of construction or assembly, the cover 32 may constitute part of the cover 12. As shown in FIG. 7, the bag 12 defines or includes the first end 14 and second end 16, where the first end 14 is open and the second end 16 is sealed as previously described. The bag 12 may further define a first portion 42 adjacent to, in proximity to, or including the first end 14, and a second portion 44 adjacent to, in proximity to, or including the second end 16. The bag 12 may optionally include a neck or tapered portion 46. Now referring to FIG. 8, the first portion 42 may be moved or positioned in the direction of the arrow to surround, circumscribe, or otherwise overlay at least a portion of the second portion 44. This configuration is similar to that shown in FIG. 1 where the cover surrounds or overlays at least a portion of the exterior surface of the bag 12. Continuing to refer to FIG. 8, moving the first portion 42 towards the second portion 44 may create or define a fold 48 where the first and second portions overlap and create a passage or opening into the interior cavity 18 formed by the second portion 44. As shown in FIG. 9, the connector 20 may then be positioned at least partially within the fold 48 such that the passage 26 of the connector 20 is in fluid communication with the interior cavity 18. The overlapped region of the first and second portions adjacent to or in proximity to the fold 48 may then be sealed to the connector 20 and/or the overmolding 30 as previously described. Once sealed to the connector 20, the first end 14 and the first portion 42 may then be moved or transitioned in the direction of the arrow shown in FIG. 7 to enclose the connector 20, and the first end 14 may be sealed to provide a sterile enclosure and configuration similar to that shown in FIG. 2. The fold 48 and/or overlapped segments of the first and second portions 42, 44 may be attached to one or more of an interior, exterior, and/or overmolding of the connector 20, as described herein.

In an exemplary method of use, the container 10 may be used to store, sterilize, and/or transfer one or more products, components, or other items. For example, the container 10 may be substantially in the configuration as shown in FIG. 1, with the first end 14 of the bag 12 sealed to the connector 20, and the first end 34 of the cover 32 also sealed to the connector 20 at one end. In this configuration, the second end 36 of the cover 32 is independently movable and positionable about an exterior surface of the bag 12 and the connector 20. The second end 36 of the cover 32 is moved to enclose the second end 24 of the connector, and is sealed to provide a leak-tight cavity 40 therein.

One or more sterilizable components may be into the interior cavity 18 of the bag 12. The sterilizable components may be introduced in several different ways. In one non-limiting example, one or more sterilizable components may be introduced through the second end 24 of the connector 20, through passage 26 and/or through the sleeve 31 and into the interior cavity 18. The cover 32 may be sealed around the connector 20 after filling the bag 12. In another example, one or more sterilizable components may be introduced into the cavity 18 through an opening (not shown) in the second end 16 of the bag 12, with the opening sealed, fused, or otherwise closed after the desired contents are inside the bag 12.

Once the desired volume or quantity of components is in the interior cavity 18, and the second end 36 of the cover 32 has been sealed to enclose the second end 24 of the connector, the container 10, and thus the sealed contents therein, may then be sterilized by any appropriate modality, e.g., gamma irradiation, steam, ultraviolet exposure, etc. The sealed cover 32 cannot be readily detached, inadvertently opened, or otherwise compromised as is the case with covers elastically mounted onto a container. The contents and the connector 20 are thus protected against unwanted contamination until the container 10 is subsequently opened.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A container, comprising:
a bag defining a first end, a second end, an exterior surface, and an interior cavity;
a connector defining a first end, a second end, and a passage therethrough, wherein the first end of the connector is attached to the first end of the bag, and the passage is in fluid communication with the interior cavity; and
a cover attached to at least one of the bag and the connector, wherein the cover is transitionable from a first configuration where the cover surrounds at least a portion of the exterior surface of the bag, to a second configuration where the cover encloses the second end of the connector,
wherein an overmolding connects at least a portion of the connector with at least one of the cover and the bag.

2. The container of claim 1, wherein the bag is made of at least one of polyethylene (PE), high-density polyethylene (HDPE), polypropylene (PP), or polyvinylidene fluoride (PVDF).

3. The container of claim 1, wherein the cover is made of at least one of polyethylene (PE), high-density polyethylene (HDPE), or polypropylene (PP), polyvinylidene fluoride (PVDF).

4. The container of claim 1, wherein the connector is made of at least one of polycarbonate (PC), acrylonitrile butadiene styrene (ABS), polyvinylidene fluoride (PVDF), polybutylene terephthalate (PBT).

5. The container of claim 1, wherein the overmolding is made of at least one of polyethylene (PE) or polypropylene (PP).

6. The container of claim 1, wherein the bag is attached to an inner perimeter of the first end of the connector.

7. The container of claim 6, wherein the cover is attached to an exterior perimeter of the connector.

8. The container of claim 1, wherein the bag is attached to an exterior perimeter of the first end of the connector.

9. The container of claim 8, wherein the cover is attached to an exterior perimeter of the first end of the bag.

10. The container of claim 1, wherein the overmolding connects the connector to at least one of the cover and the bag as a 360° circumferential seal about the connector.

11. The container of claim 1, wherein the cover is a portion of the bag folded over itself.

12. The container of claim 1, wherein the cover defines a first end sealed to at least one of the bag and the connector, and the cover defines a second end independently movable about the connector.

13. A method for producing a container, comprising:
providing a bag having a first end, a second end, and a cavity therein;
forming a fold in the bag such that a first portion of the bag covers at least a portion of an exterior surface of a second portion of the bag; and
securing at least a portion of the fold to a connector configured to be connected to a chamber, the connector defining a passage in fluid communication with the cavity of the bag.

14. The method of claim 13, wherein forming the fold includes positioning the first portion of the bag to circumscribe at least a portion of the exterior surface of the second portion.

15. The method of claim 13, wherein the first portion of the bag is sized to enclose the connector therein.

16. The method of claim 13, further comprising manipulating the first portion of the bag to enclose the connector therein, and sealing the first portion of the bag.

17. The method of claim 16, further comprising sterilizing the first and second portions of the bag.

18. A method of sterilizing one or more components, comprising:
moving one or more components into an interior cavity of a bag attached to a connector;
enclosing the connector within a cover, wherein the cover includes a first end sealed to the connector, and a second end independently movable about the connector;
sealing the second end of the cover to contain the connector therein; and
sterilizing the connector within the cover and the one or more components within the bag.

19. The method of claim 18, wherein sterilizing the connector and the one or more components is performed using at least one of ethylene oxide, gamma radiation, or x-ray radiation.

20. The method of claim 18, wherein sterilizing the connector and the one or more components is performed using steam.
